# EUROPÄISCHE PATENTANMELDUNG

(11) **EP 3 225 218 A1**
(43) Veröffentlichungstag der Anmeldung: **04.10.2017**
(21) Anmeldenummer: 16162862.3
(22) Anmeldetag: 30.03.2016
(51) Int. Cl.: A61F 2/966, A61M 25/00, A61F 2/24

(54) **KATHETEREINRICHTUNG MIT EINER ÜBER LASCHEN AN DEN AUSSENSCHAFT ANGEBUNDENEN IMPLANTATKAPSEL**

(71) Anmelder: Biotronik AG, 8180 Bülach (CH)
(72) Erfinder: Keller, Mark, 5000 Aarau (CH)
(74) Vertreter: Galander, Marcus

(57) **Zusammenfassung**

Die Erfindung betrifft eine Kathetereinrichtung (100) zum Transportieren eines Implantats an einen Zielort in einem Körperlumen sowie zum Freisetzen des Implantats am Zielort, mit: einem Außenschaft (5) zum Transportieren des Implantats an den Zielort, und einer Implantatkapsel (200) zum Aufnehmen des Implantats, wobei die Implantatkapsel (200) einen rohrförmigen Kapselkern (201) aufweist, der das Implantat vor dem Freisetzen umgibt. Erfindungsgemäß ist vorgesehen, dass der Kapselkern (201) an einem proximalen Ende (201a) des Kapselkerns zum Festlegen des Kapselkerns (201) am Außenschaft (5) eine Mehrzahl an Laschen (203) aufweist, die entlang einer axialen Richtung (A) des Kapselkerns (201) von einem rohrförmigen Abschnitt (202) des Kapselkerns (201) abstehen.

## Beschreibung

Die Erfindung betrifft eine Kathetereinrichtung gemäß Anspruch 1.

Derartige Kathetereinrichtungen dienen zum Transportieren eines Implantats, z. B. in Form eines Stents oder einer (z. B. bioprosthetischen) Herzklappe an einen Zielort in einem Körperlumen eines menschlichen oder ggf. tierischen Patienten. Eine solche Kathetereinrichtung weist dabei einen Innenschaft um Tragen des besagten Implantats auf, einen Außenschaft zum Transportieren des Implantats an den Zielort, wobei der Außenschaft den Innenschaft im Querschnitt umgibt. Weiterhin ist am Außenschaft eine Implantatkapsel zum Aufnehmen des Implantats vorgesehen, wobei die Implantatkapsel einen rohrförmigen Kapselkern aufweist, der das Implantat vor dem Freisetzen umgibt. Das Implantat kann durch Verschieben des Innenschaftes gegenüber dem Außenschaft aus der Implantatkapsel herausbewegt und am Zielort freigesetzt bzw. implantiert werden. Weiterhin kann ein äußerster Stabilisierungsschaft zum Stabilisieren des Außenschaftes vorgesehen sein, wobei der Außenschaft verschieblich in einem vom Stabilisierungsschaft umgebenen Lumen des Stabilisierungsschaftes angeordnet ist.

Die Implantatkapsel weist in der Regel einen Kapselkern auf, der z. B. eine Tragstruktur der Implantatkapsel bildet und am Außenschaft geeignet festzulegen ist.

Hierzu sind Lösungen bekannt, bei der ein Verbindungsmittel (Konnektor) in Form eines Kunststoff-Spritzgussteils am Außenschaft befestigt ist, an dem die Implantat- oder Prothesenkapsel aufgeklebt ist. Auch sind Lösungen bekannt, bei denen die Implantatkapsel einen Metallkern aufweist und deren Ende fingerähnliche Ausläufer hat, welche dann in einem Kunststoffkonnektor vergossen sind.

Diese, auf Klebungen, Spritzgießen etc. basierende Verbindungen sind oft sehr biegesteif und weisen eine große Baulänge auf, was zu einer inhomogenen Biegeform des Katheters führt. Zudem zeigt der Katheter in diesem Übergangsbereich einen ungünstigen Steifigkeitssprung auf.

Hiervon ausgehend liegt der vorliegenden Erfindung die Aufgabe zu Grunde, eine Kathetereinrichtung der eingangs genannten Art hinsichtlich der vorgenannten Problematik weiter zu verbessern.

Diese Aufgabe wird durch eine Kathetereinrichtung mit den Merkmalen des Anspruchs 1 gelöst.

Vorteilhafte Ausgestaltungen der Erfindung sind in den Unteransprüchen angegeben und werden nachfolgend beschrieben.

Erfindungsgemäß sieht der Gegenstand des Anspruchs 1 vor, dass der Kapselkern an einem Ende des Kapselkerns zum Festlegen des Kapselkerns am Außenschaft eine Mehrzahl an Laschen aufweist, die entlang einer axialen Richtung des Kapselkerns von einem rohrförmigen Abschnitt des Kapselkerns abstehen.

Dies erlaubt eine Verbindung des Kapselkerns zum Außenschaft über die besagten Laschen, die mit Vorteil eine ausreichende mechanische Stabilität bereitstellt und eine vergleichsweise kurze Baulänge ermöglicht, bei der der Anteil des steifen Abschnittes vorteilhaft kurz gehalten werden kann.

Im Sinne der vorliegenden Erfindung bedeutet distal, dass eine entsprechende distale Komponente, ein distaler Abschnitt oder ein distales Ende in der axialen Richtung des Außenschaftes, entlang der sich die Längsachse des Außenschaftes erstreckt, weiter von einem Handgriff bzw. einem Bediener (Arzt) der Kathetereinrichtung entfernt ist, als eine proximale Komponente, ein proximaler Abschnitt bzw. ein proximales Ende.

Die Kathetereinrichtung kann weiterhin einen im Außenschaft verschieblich gelagerten Innenschaft zum Tragen des Implantats aufweisen, wobei das Implantat durch Verschieben des Außenschaftes gegenüber dem Innenschaft aus der Implantatkapsel herausführbar ist.

Die Erfindung eignet sich insbesondere für ein Kathetersystem, wo das Implantat durch eine Verschiebung des Außenschaftes gegenüber dem Innenschaft freigesetzt wird, insbesondere erfolgt die Freisetzung durch Zurückziehen des Außenschaftes. In der bevorzugten Variante weist dann der Kapselkern an einem proximalen Ende des Kapselkerns eine Mehrzahl von Laschen zum Festlegen des Kapselkerns am Außenschaft auf, wobei die Laschen entlang einer axialen Richtung des Kapselkerns von einem rohrförmigen Abschnitt des Kapselkerns abstehen.

Die Implantatkapsel kann weiterhin eine äußere, auf dem Kapselkern angeordnete Schicht bzw. Materiallage aufweisen, die vorzugsweise aus einem elastischen oder viskoelastischen Polymer besteht. Als Material kommen hier Polymer wie Polyurethan oder thermoplastische Polymere wie thermoplastische Copolyamide, wie beispielsweise unter dem Handelsnamen PEBAX bekannt, in Frage.

Weiterhin kann die Implantatkapsel eine innere vom Kapselkern umgebene und daran festgelegte Schicht bzw. Materiallage aufweisen, die das eigentliche Implantat umgibt und vorzugsweise aus einem Kunststoff mit reduzierten Reibungskoeffizienten besteht. Insbesondere eignet sich hier PTFE (auch unter dem Handelsnamen Teflon bekannt) oder ePT-FE bzw. ein Kunststoffverbund mit Anteilen aus PTFE oder ePTFE.

Es ist aber auch denkbar, eine einlagige Implantatkapsel zu verwenden, die in diesem Falle nur aus dem Kapselkern bestehen würde, der dann als Kapselhülle bezeichnet wird. In einer derartigen Ausgestaltung ist ein Kompositmaterial aus Kohlefasern und einem Kunststoff vorteilhaft, wobei die Kohlefasern in den Kunststoff integriert sind.

Gemäß einer bevorzugten Ausführungsform der Erfindung ist weiterhin vorgesehen, dass die Laschen jeweils zwei entlang der axialen Richtung erstreckte Seiten aufweisen, die in einer Umfangsrichtung des rohrförmigen Abschnitts einander abgewandt sind, wobei die beiden Seiten ausgehend von dem besagten rohrförmigen Abschnitt entlang der axialen Richtung auseinanderlaufen, so dass sich die jeweilige Lasche (ausgehend vom besagten rohrförmigen Abschnitt) entsprechend verbreitert. Die Laschen werden daher auch als Schwalbenschwänze bezeichnet. In dieser Ausgestaltung kommt es darauf an, dass die beiden Seiten ausgehend von dem rohrförmigen Abschnitt im Wesentlichen auseinander laufen. Die Laschen können jedoch kleine Unterbrechungen oder Einkerbungen aufweisen, ohne dass die Funktion dieser Ausgestaltung beeinträchtigt wird. Entscheidend ist die Verbreiterung für eine gute Kraftübertragung.

Weiterhin ist gemäß einer Ausführungsform der Erfindung vorgesehen, dass an einem distalen Endabschnitt des Außenschaftes ein den Außenschaft vorzugsweise umlaufendes Verbindungsmittel festgelegt ist, insbesondere festgeschweißt ist, über das die Laschen mit dem Außenschaft verbunden sind, und zwar vorzugsweise durch Verschrauben der Laschen mit dem Verbindungsmittel. Verschrauben ist die bevorzugte und einfachste Art der Befestigung. Sie bietet auch zusätzlich den mechanischen Vorteil, dass die Vorspannung der Laschen präzise in Zusammenspiel mit dem Verbindungsmittel festgelegt werden kann. Bei einer entsprechenden Ausfräsung des Verbindungsmittels können die seitlichen Flanken der Laschen spielfrei in die Ausfräsung eingebracht werden. Die Lasche wird über die Verschraubung gespannt und die Flanken liegen spielfrei an den entsprechenden Gegenstücken der Aussparung. Prinzipiell sind jedoch genauso andere Befestigungsarten wie Verkleben oder Verschweißen der Laschen denkbar, jedoch ist hier die Erzeugung der Vorspannung und das spielfreie Einbringen der Laschen in eine mögliche Ausfräsung im Verbindungsmittel aufwendiger zu erzeugen.

Besonders bevorzugt ist das Verbindungsmittel, das vorliegend auch als Konnektor bezeichnet wird, als ein ringförmiger Körper ausgebildet, der den Außenschaft im Querschnitt umgibt. In einer Ausgestaltung der Erfindung, wo der Katheter über eine antegrade Route eingeführt wird, umgibt der Konnektor den Innenschaft im Querschnitt.

Weiterhin ist gemäß einer bevorzugten Ausführungsform der Erfindung vorgesehen, dass die jeweilige, am Verbindungsmittel festgelegte Lasche in eine zugeordnete Ausnehmung des Verbindungsmittels eingreift, wobei diese Ausnehmungen an einer umlaufenden Außenseite des Verbindungsmittels ausgebildet sind. Bevorzugt ist gemäß einer Ausführungsform der Erfindung vorgesehen, dass die jeweilige, am Verbindungsmittel festgelegte Lasche formschlüssig in die jeweils zugeordnete Ausnehmung des Verbindungsmittels eingreift.

Weiterhin ist gemäß einer bevorzugten Ausführungsform der Erfindung vorgesehen, dass die besagte Außenseite an einem proximalen Ende des Verbindungsmittels einen umlaufenden konischen Abschnitt aufweist, so dass das Verbindungsmittel an dem proximalen Ende des Verbindungsmittels eine umlaufende Fase an der Außenseite aufweist, wobei jene Ausnehmungen zur Aufnahme der Laschen in dem konischen Abschnitt der Außenseite ausgebildet sind.

Weiterhin ist gemäß einer bevorzugten Ausführungsform der Erfindung vorgesehen, dass die jeweilige Ausnehmung zwei einander zugwandte Flanken aufweist, die von einem Boden der jeweiligen Ausnehmung abgehen, wobei sich die Ausnehmungen in axialer Richtung des Außenschaftes zum rohrförmigen Abschnitt des Kapselkerns hin verjüngen, so dass die jeweiligen beiden Flanken entsprechend aufeinander zu laufen.

Weiterhin ist gemäß einer bevorzugten Ausführungsform der Erfindung vorgesehen, dass die jeweilige Lasche in einem nicht an dem Verbindungsmittel festgelegten Zustand eine Krümmung in der Umfangsrichtung des rohrförmigen Abschnitts aufweist. Diese Krümmung kann der Krümmung des rohrförmigen Abschnitts in dessen Umfangsrichtung entsprechen.

Weiterhin ist gemäß einer bevorzugten Ausführungsform der Erfindung vorgesehen, dass die jeweilige an dem Verbindungsmittel festgelegte Lasche eine geringere Krümmung in der Umfangsrichtung als in dem nicht an dem Verbindungsmittel festgelegten Zustand aufweist, wobei die jeweilige an dem Verbindungsmittel festgelegte Lasche mit jeder ihrer Seiten gegen eine zugeordnete Flanke der Ausnehmung drückt, in die die Lasche eingreift.

Mit anderen Worten wird die jeweilige Lasche beim Festlegen, insbesondere Festschrauben in der jeweiligen Ausnehmung flachgedrückt, wobei der Kontakt der Außenseiten der jeweiligen Lasche zu den Flanken der zugeordneten Ausnehmung hergestellt wird.

Weiterhin ist gemäß einer bevorzugten Ausführungsform der Erfindung vorgesehen, dass die besagte geringere Krümmung nicht verschwindet. D. h., dass die Laschen so in den Ausnehmungen festgelegt bzw. festgeschraubt werden, dass sie nicht restlos flachgepresst werden, sondern eine gewisse Restkrümmung bestehen bleibt.

Weiterhin ist gemäß einer bevorzugten Ausführungsform der Erfindung vorgesehen, dass der rohrförmige Abschnitt mit zwischen benachbarten Laschen verlaufenden Randabschnitten an einer umlaufenden Stufe des Verbindungsmittels anliegt, wobei gemäß einer bevorzugten Ausführungsform der Erfindung vorgesehen ist, dass die besagten Randabschnitte durch die am Verbindungsmittel festgelegten Laschen gegen die Stufe gezogen werden. Dies kann durch das Auseinanderlaufen der Außenseiten der Laschen sowie der entsprechenden Form der Flanken sichergestellt werden, wodurch bewirkt wird, dass die Laschen den daran festgelegten rohrförmigen Abschnitt des Kapselkerns beim Festlegen gegen die Stufe ziehen.

Weiterhin ist gemäß einer bevorzugten Ausführungsform der Erfindung vorgesehen, dass die am Verbindungsmittel festgelegten Laschen ausgehend vom rohrförmigen Abschnitt zum Boden der jeweils zugeordneten Ausnehmung des Verbindungsmittels hin gebogen sind.

Weiterhin ist gemäß einer bevorzugten Ausführungsform der Erfindung vorgesehen, dass die Laschen mittels Schrauben am Verbindungselement festgelegt sind, wobei die jeweilige Schraube durch eine Durchgangsöffnung der jeweiligen Lasche greift und mit ihrem Außengewinde mit einem Innengewinde einer zugeordneten Öffnung im Boden der jeweiligen Ausnehmung verschraubt ist.

Weiterhin ist gemäß einer bevorzugten Ausführungsform der Erfindung vorgesehen, dass der Kapselkern und/oder das Verbindungsmittel aus einem Metall, insbesondere aus einem Stahl gefertigt ist bzw. sind. Vorteilhafterweise kommen als Materialien in dieser Ausführungsform der Erfindung neben rostfreien Stahl, Chrom-Nickel Stahl auch Cobalt-Chrom Gemische oder Legierungen in Frage. Ebenso zweckmäßig sind in bestimmten Ausgestaltungen auch Nickel-Titan Legierungen (Nitinol) oder Verbundwerkstoffe mit Kohlefasern.

Weiterhin ist gemäß einer bevorzugten Ausführungsform der Erfindung vorgesehen, dass der rohrförmige Abschnitt des Kapselkerns eine Mehrzahl an parallelen sowie entlang der Umfangsrichtung verlaufenden Schlitzen sowie insbesondere zwischen benachbarten Schlitzen angeordnete Durchgangsöffnungen aufweist. Die Durchgangsöffnungen entstehen dabei in bestimmten Ausgestaltungen der Erfindung, wo die Schlitze mittels Laserschneiden in einem Kapselkern aus Metall erzeugt werden, automatisch als Durchschmelzlöcher. Entscheidend für diese Ausgestaltung sind jedoch die in Umfangsrichtung verlaufenden Schlitze. Durch die Breite und Häufigkeit der Schlitze kann die Biegesteifigkeit des Kapselkerns variiert werden. Dabei wird die Biegesteifigkeit des Kapselkerns bevorzugt zum Übergang zum Verbindungsmittel bzw. Konnektor hin graduell erhöht, um zu gewährleisten, dass die infolge Biegebeanspruchung auftretenden Zug- und Druckspannungen kontinuierlich in diesen eingeleitet werden.

Weitere Merkmale und Vorteile der Erfindung sollen bei der Figurenbeschreibung von Ausführungsbeispielen der Erfindung anhand der Figuren erläutert werden. Es zeigen:
- Fig. 1: eine ausschnitthafte Ansicht einer erfindungsgemäßen Kathetereinrichtung,
- Fig. 2: eine ausschnitthafte perspektivische Ansicht eines Kapselkerns vor dem Festlegen am Verbindungsmittel (Konnektor);
- Fig. 3: eine weitere ausschnitthafte perspektivische Ansicht eines Kapselkerns vor dem Festlegen am Verbindungsmittel (Konnektor);
- Fig. 4: eine ausschnitthafte perspektivische Ansicht eines am Verbindungsmittel festgelegten Kapselkerns; und
- Fig. 5: eine Darstellung der beim Festlegen auftretenden Kräfte.

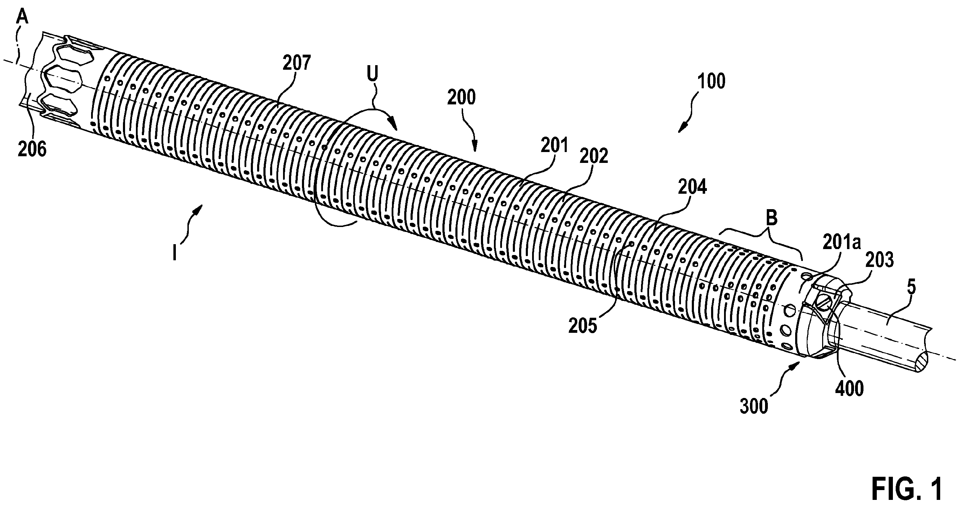

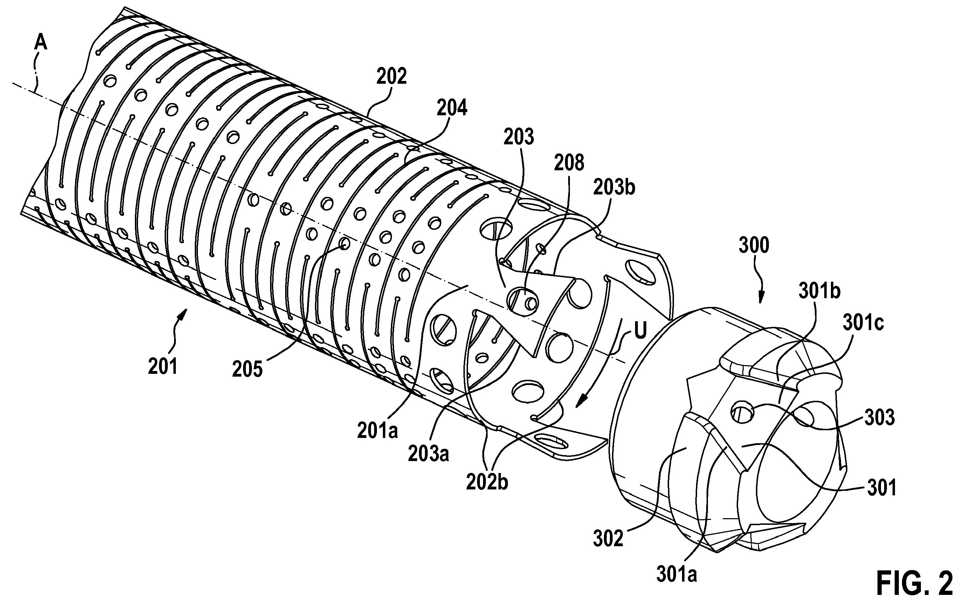

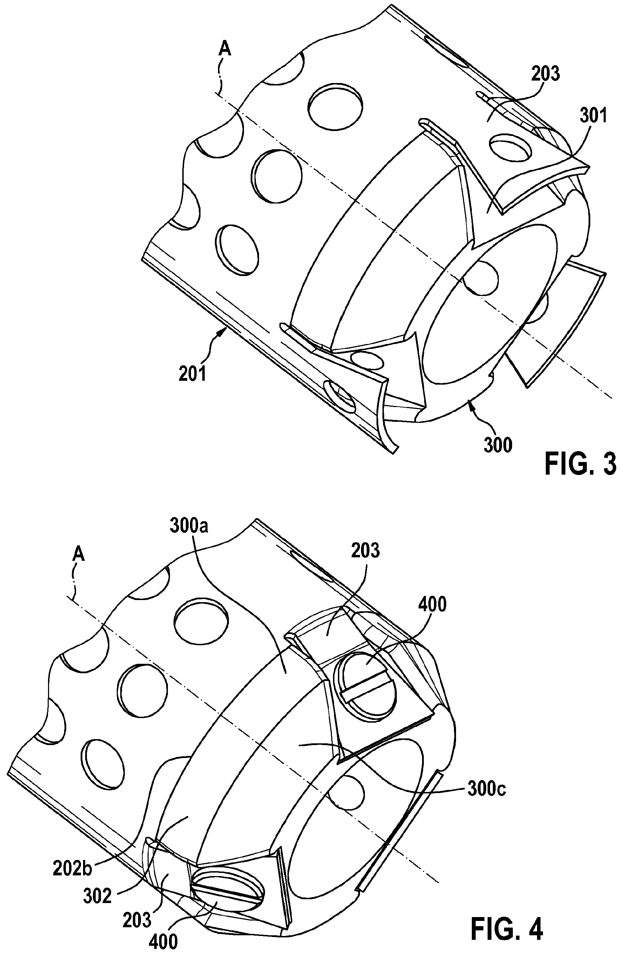

Figur 1 zeigt im Zusammenhang mit den Figuren 2 bis 4 eine Kathetereinrichtung 100 zum Transportieren eines Implantats I (nicht gezeigt) an einen Zielort eines Körperlumens eines Patienten. Die Einrichtung 100 weist hierbei einen längs erstreckten Innenschaft (nicht gezeigt) zum Tragen des Implantats I auf, wobei das Implantat I an einem distalen Ende des Innenschaftes festlegbar ist. Das distale Ende des Innenschaftes liegt seinem proximalen Ende gegenüber, das z. B. an einem Griff der Kathetereinrichtung festgelegt sein kann, über den der Innenschaft sowie der Außenschaft 5 bewegt werden. Der Außenschaft 5 dient dabei zum Transportieren des Implantats an den gewählten Zielort im Körperlumen des Patienten. Hierbei umgibt der Außenschaft 5 den Innenschaft, so dass dieser im Außenschaft 5 führbar ist und gegenüber dem Außenschaft 5 bewegbar ist, um z. B. in bekannter Weise das Implantat aus einer am Außenschaft 5 festgelegten Implantatkapsel 200 herauszubewegen, so dass sich dieses am Zielort entfalten kann bzw. dort entfaltbar ist. In dieser Ausgestaltung weist auch der Außenschaft 5 einen Kern aus einem geschnittenen Metallrohr (auch als Hypotube bezeichnet) auf, welcher mit einem Polymer ummantelt ist (nicht dargestellt).

Die Implantatkapsel 200 weist einen Kapselkern 201 aus einem Metall auf, z. B. aus einem Stahlrohr, das so geschnitten ist, dass es an seinem proximalem Ende 201a auslaufende Laschen 203 aufweist, die ein z. B. trapezförmiges Ende ausbilden. Somit weist jede Lasche 203 bzw. jeder Schwalbenschwanz 203 voneinander abgewandte Außenseiten 203a und 203b auf, die ausgehend von einem rohrförmigen Abschnitt 202 des Kapselkerns 201 auseinander laufen. Der Kapselkern 201 kann weiterhin nach außen hin von einer äußeren Schicht oder Materiallage 207 (auch als Outer Jacket bezeichnet) umgeben sein, wobei an der Innenseite des Kapselkerns eine innere Schicht oder Materiallage 206 vorgesehen sein kann, die auch als Inner Liner bezeichnet wird.

Das Stahlrohr bzw. der rohrförmige Abschnitt 202 weist ein Schnittmuster auf, das z. B. parallele sowie in einer Umfangsrichtung U verlaufende Schlitze 204 und zwischen den Schlitzen 204 angeordnete Durchgangsöffnungen 205 aufweist. Das Schnittmuster ist dabei so gestaltet, dass die Biegesteifigkeit des Kapselkerns 201 am Übergang zu einem Konnektor 300, an dem die Laschen 203 festgelegt sind, graduell anwächst.

So wird gewährleistet, dass die infolge einer Biegebeanspruchung auftretenden Zug- und Druckspannungen kontinuierlich in den Konnektor 300 eingeleitet werden.

Der bevorzugt aus einem Metall gefertigte ringförmige Konnektor bzw. Verbindungsmittel 300 weist bevorzugt eine Außenseite 300a mit einem konischen Abschnitt 300c auf Auf dieser Kegelfläche 300c des Konus sind vorzugsweise drei Schwalbenschwanz- bzw. trapezförmige Ausnehmungen 301 eingearbeitet, welche sich zum distalen Ende der Kathetereinrichtung 100 hin verjüngen. In diese Taschen 301 werden die formkongruenten Gegenstücke in Form der Laschen 203, welche sich an der Kapsel befinden und vorzugsweise integral mit einem proximalen Ende 201a des Kapselkerns 201 ausgebildet sind, mittels je einer Schraube 400 hineinmontiert. Hierbei durchgreift die jeweilige Schraube 400 eine Durchgangsöffnung 208 der zugeordneten Lasche 203 und ist in eine am Boden 301c der jeweiligen Ausnehmung 301 vorgesehene Öffnung 303 eingeschraubt. Da diese Gegenstücke 203 in der Umfangsrichtung U gekrümmt sind, werden diese beim Herunterschrauben flachgedrückt. Dies hat zur Folge, dass sich die Außenseiten 203a, 203b der Laschen gegen die entsprechend verlaufenden Flanken bzw. Wände 301a, 301b der Ausnehmungen 301 pressen. Es entsteht dabei eine vorgespannte formschlüssige Verbindung, welche insbesondere spielfrei ist. Der Konnektor 300 weist zudem eine umlaufende Stufe oder Ring 302 auf, welcher als Anschlag dient, wobei die zwischen den Laschen 203 sich erstreckenden Randabschnitte 201b des rohrförmigen Abschnitts 202 des Kapselkerns 201 gegen jenen Anschlag gezogen werden.

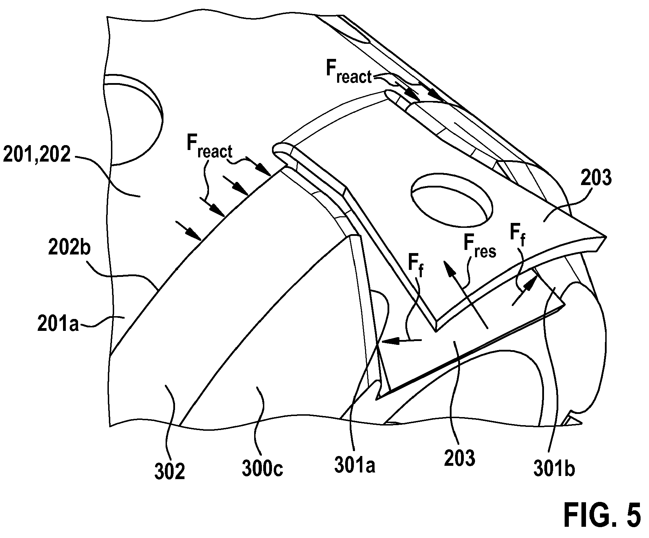

Die beim Festlegen der Laschen 203 am Verbindungsmittel/Konnektor 300 auftretenden Kräfte sind in der Figur 5 angedeutet.

Durch das Herunterdrücken der gebogenen Lasche (z.B. Schwalbenschwanz) 203 begradigt sich diese in radialer Richtung bzw. Umfangsrichtung U und drückt mit F_{f} an die Flanken 301a, 301b des Konnektors 300.

Die resultierende Kraft Fᵣₑₛ zieht als Folge den Kapselkern 201 mit seinen Randabschnitten 202b an die Stufe oder Anschlagschulter 302 am Konnektor 300. Der Kapselkern 201 drückt sich mit den Reaktionskräften F_{react} an diese an.

Die Geometrie der Laschen und des jeweiligen Gegenstücks im Konnektor 300 (Ausnehmungen 301) sind so gewählt, dass der Bogen im montierten Zustand immer ein wenig erhalten bleibt; so kann garantiert werden, dass die Bauteile gegeneinander verspannt sind und somit die Kontaktflächen aneinander aufliegen.

Die Kapsel 200 wird auf die vorstehend beschriebene Weise mit ihrem kräftetragenden Element, dem z. B. aus einem Metallrohr lasergeschnittenen Kapselkern 201, im Ergebnis formschlüssig und spielfrei mit dem Außenschaft 5 verbunden. Gemäß einer Ausführungsform ist der distale Konnektor 300 aus einem Stahl gefertigt, wobei er katheterseitig bevorzugt auf den Außenschaft geschweißt ist. Die hohe Belastbarkeit des Stahles am Konnektor 300 und in der Kapsel 200 lassen so eine kurze Bauweise zu, was den biegesteifen Anteil des Katheterendes wesentlich verkürzt. Mit der spielfreien Verbindung zum Konnektor 300 und einem zusätzlichen Verschweißen der äußeren Kunststoffschichten wird zudem sichergestellt, dass die Dichtigkeit im Übergang gewährleistet ist. Alternativ kann auch ein O-Ring zur Herstellung der Dichtigkeit verwendet werden (nicht dargestellt). Ein weiterer Vorteil dieser Bauweise ist, dass der Fertigungsprozess kein Spritzgießen und Kleben beinhaltet. Dies erlaubt es, die Kapsel 200 mit einfachen Schrumpfschläuchen und Heißluftgebläse im Schmelzverfahren herstellen zu können, bzw. das Outer Jacket 207 aufbringen zu können und über Durchschmelzlöcher (z. B. 205) im Kapselkern 201 den Inner Liner 206 am Kern 201 fixieren zu können.

## Patentansprüche

1. Kathetereinrichtung (100) zum Transportieren eines Implantats (I) an einen Zielort in einem Körperlumen sowie zum Freisetzen des Implantats am Zielort, mit:
- einem Außenschaft (5) zum Transportieren des Implantats an den Zielort,
- einer Implantatkapsel (200) zum Aufnehmen des Implantats, wobei die Implantatkapsel (200) einen rohrförmigen Kapselkern (201) aufweist, der das Implantat vor dem Freisetzen umgibt,
**dadurch gekennzeichnet,**
**dass** der Kapselkern (201) an einem Ende (201a) des Kapselkerns zum Festlegen des Kapselkerns (201) am Außenschaft (5) eine Mehrzahl an Laschen (203) aufweist, die entlang einer axialen Richtung (A) des Kapselkerns (201) von einem rohrförmigen Abschnitt (202) des Kapselkerns (201) abstehen.

2. Kathetereinrichtung nach Anspruch 1, **dadurch gekennzeichnet, dass** die Laschen (203) jeweils zwei entlang der axialen Richtung erstreckte Seiten (203a, 203b) aufweisen, wobei die beiden Seiten (203a, 203b) ausgehend von dem besagten rohrförmigen Abschnitt (202) entlang der axialen Richtung (A) auseinanderlaufen, so dass sich die jeweilige Lasche (203) entsprechend verbreitert.

3. Kathetereinrichtung nach Anspruch 1 oder 2, **dadurch gekennzeichnet, dass** an einem distalen Endabschnitt (5a) des Außenschaftes (5) ein Verbindungsmittel (300) festgelegt ist, über das die Laschen (203) mit dem Außenschaft (5) verbunden sind, vorzugsweise verschraubt sind.

4. Kathetereinrichtung nach Anspruch 3, **dadurch gekennzeichnet, dass** die jeweilige am Verbindungsmittel (300) festgelegte Lasche (203) in eine zugeordnete Ausnehmung (301) des Verbindungsmittels (300) eingreift, wobei diese Ausnehmungen (301) an einer umlaufenden Außenseite (300a) des Verbindungsmittels (300) ausgebildet sind.

5. Kathetereinrichtung nach Anspruch 4, **dadurch gekennzeichnet, dass** die Außenseite (300a) an einem proximalen Ende (300b) des Verbindungsmittels (300) einen konischen Abschnitt (300c) aufweist, wobei jene Ausnehmungen (301) in dem konischen Abschnitt (300c) der Außenseite (300a) ausgebildet sind.

6. Kathetereinrichtung nach Anspruch 4 oder 5, **dadurch gekennzeichnet, dass** die jeweilige Ausnehmung (301) zwei einander zugwandte Flanken (301a, 301b) aufweist, die von einem Boden (301c) der jeweiligen Ausnehmung (301) abgehen, wobei sich die Ausnehmungen (301) entlang der axialen Richtung (A) des Außenschaftes (5) zum rohrförmigen Abschnitt (202) des Kapselkerns (201) hin verjüngen, so dass die jeweiligen beiden Flanken (301a, 301b) entsprechend aufeinander zu laufen.

7. Kathetereinrichtung nach einem der Ansprüche 3 bis 6, **dadurch gekennzeichnet, dass** die jeweilige Lasche (203) in einem nicht an dem Verbindungsmittel (300) festgelegten Zustand eine Krümmung in der Umfangsrichtung (U) des rohrförmigen Abschnitts (202) aufweist.

8. Kathetereinrichtung nach den Ansprüchen 2, 6 und 7, **dadurch gekennzeichnet, dass** die jeweilige an dem Verbindungsmittel (300) festgelegte Lasche (203) eine geringere Krümmung in der Umfangsrichtung (U) als in dem nicht an dem Verbindungsmittel (300) festgelegten Zustand aufweist, wobei die jeweilige an dem Verbindungsmittel (300) festgelegte Lasche (203) mit jeder ihrer Seiten (203a, 203b) gegen eine zugeordnete Flanke (301a, 301b) sowie den Boden (301c) der Ausnehmung (301) drückt, in die die Lasche (203) eingreift.

9. Kathetereinrichtung nach Anspruch 8, **dadurch gekennzeichnet, dass** die besagte geringere Krümmung nicht verschwindet.

10. Kathetereinrichtung nach einem der Ansprüche 3 bis 9, **dadurch gekennzeichnet, dass** der rohrförmige Abschnitt (202) mit zwischen benachbarten Laschen (203) verlaufenden Randabschnitten (202b) an einer umlaufenden Stufe (302) des Verbindungsmittels anliegt.

11. Kathetereinrichtung nach Anspruch 10, **dadurch gekennzeichnet, dass** die besagten Randabschnitte (202b) durch die am Verbindungsmittel (300) festgelegten Laschen (203) gegen die Stufe (302) gezogen werden.

12. Kathetereinrichtung nach Anspruch 4 oder einem der Ansprüche 5 bis 11 soweit rückbezogen auf Anspruch 4, **dadurch gekennzeichnet, dass** die am Verbindungsmittel (300) festgelegten Laschen (203) ausgehend vom rohrförmigen Abschnitt (202) zum Boden (301c) der jeweils zugeordneten Ausnehmung (301) des Verbindungsmittels (300) hin gebogen sind.

13. Kathetereinrichtung nach einem der Ansprüche 3 bis 12, **dadurch gekennzeichnet, dass** die Laschen (203) mittels Schrauben (400) am Verbindungselement (300) festgelegt sind.

14. Kathetereinrichtung nach einem der vorhergehenden Ansprüche, **dadurch gekennzeichnet, dass** der Kapselkern (201) und/oder das Verbindungsmittel (300) aus einem Metall, insbesondere aus einem Stahl gefertigt sind.

15. Kathetereinrichtung nach einem der vorhergehenden Ansprüche, **dadurch gekennzeichnet, dass** der rohrförmige Abschnitt (202) des Kapselkerns (201) eine Mehrzahl an parallelen sowie entlang der Umfangsrichtung verlaufenden Schlitzen (204) sowie insbesondere zwischen benachbarten Schlitzen (204) angeordnete Durchgangsöffnungen (205) aufweist.
